Europäisches Patentamt

European Patent Office

Office européen des brevets

(19)

(11) Numéro de publication: **0 098 983**
**B1**

(12) **FASCICULE DE BREVET EUROPEEN**

(45) Date de publication du fascicule du brevet:
28.12.88

(21) Numéro de dépôt: **83105783.1**

(22) Date de dépôt: **13.06.83**

(51) Int. Cl.⁴: **A 41 B 13/02**

(54) **Couche-culotte perfectionnée.**

(30) Priorité: **24.06.82 FR 8211107**

(43) Date de publication de la demande:
**25.01.84 Bulletin 84/4**

(45) Mention de la délivrance du brevet:
**28.12.88 Bulletin 88/52**

(84) Etats contractants désignés:
**AT BE CH DE GB IT LI**

(56) Documents cités:
**DE-A- 1 441 365**
**DE-A- 1 924 813**
**FR-A- 2 178 745**
**FR-A- 2 355 496**
**US-A- 2 551 663**
**US-A- 3 431 911**
**US-A- 3 882 871**
**US-A- 3 916 900**
**US-A- 3 955 575**
**US-A- 4 041 949**

(73) Titulaire: **PEAUDOUCE, 59, Rue de la Vignette,**
**F-59126 Linselles (FR)**

(72) Inventeur: **De Jonckheere, Raphael, 797 Domaine de la**
**Vigne, F-59910 Bondues (FR)**
Inventeur: **Dussaud, Jacques, 26, avenue du Maréchal**
**Leclerc, F-59110 La Madeleine (FR)**

(74) Mandataire: **Casalonga, Axel et al, BUREAU D.A.**
**CASALONGA - JOSSE Morassistrasse 8,**
**D-8000 Munich 5 (DE)**

ACTORUM AG

## Description

La présente invention se rapporte à une couche-culotte, notamment pour enfants en bas âge, comprenant une enveloppe extérieure pratiquement étanche à l'eau, une feuille intérieure perméable à l'eau, et un coussin absorbant disposé entre ladite enveloppe extérieure et ladite feuille intérieure.

Sur les couches-culottes connues, aussi bien l'enveloppe extérieure que la feuille intérieure sont constituées par des matériaux non élastiques, par exemple l'une par une feuille de polyéthylène et l'autre par un voile de non tissé. Sur ces couches-culottes connues, on a déjà prévu de rapporter des élastiques à la taille et aux passages pour les jambes. Les élastiques à la taille ont pour fonction de permettre une meilleure tenue de la couche-culotte. Les élastiques aux passages pour les jambes ont pour fonction d'améliorer l'étanchéité de la couche-culotte à ces endroits critiques.

Ces élastiques rapportés dont la pose complique considérablement la fabrication des couches-culottes ne donnent néanmoins pas entière satisfaction. En effet, outre le serrage excessif qu'ils peuvent provoquer ces élastiques disposés uniquement aux bords de la couche-culotte provoque fréquemment un gonflement inesthétique des parties restantes de la couche-culotte. De plus, l'efficacité de ces élastiques rapportés dépend dans une large mesure de la précision avec laquelle la couche-culotte a été posée sur l'enfant ce qui nécessite une habileté qui ne peut, dans le meilleur des cas, être acquise qu'au bout d'un certain temps.

Il est en outre déjà connu, dans le domaine des articles d'hygiène, d'utiliser des enveloppes ou feuilles élastiques et/ou en mousse de matière plastique sur des articles d'hygiène.

Ainsi, le document FR-A-2 355 495 concerne des pansements, couches pour bébé, garnitures etc., avec un matelas absorbant recouvert sur une face d'une mince pellicule élastique et sur la face opposée d'une feuille de recouvrement élastique, la pellicule et la feuille pouvant être constituées par la même matière. Cet article d'hygiène n'est pas constitué par une couche-culotte, c'est-à-dire une culotte à couche incorporée, et ne suggère pas les multiples fonctions assumées sur la couche-culotte conforme à l'invention par l'enveloppe extérieure et la feuille intérieure, grâce à leur réalisation en mousse élastique de matière plastique, à savoir la perméabilité à l'eau de la feuille intérieure, l'imperméabilité à l'eau et la perméabilité à l'air de l'enveloppe extérieure et le serrage élastique à la taille et au passage pour les jambes.

Le document US-A-3 431 911 se rapporte également à une couche ou garniture absorbante avec un matelas absorbant recouvert sur la face interne d'une feuille de mousse réticulée de polymère, de préférence élastique, dont les bords latéraux sont rabattus sur la face externe du matelas et sont ici fixés, le but de ces rabats de mousse étant d'empêcher le glissement de la garniture sur son support, par exemple une alèse en matière plastique. Ce document ne concerne pas non plus une couche-culotte et ne suggère pas l'utilisation de mousse élastique de matière plastique pour l'enveloppe extérieure et la feuille intérieure d'une couche-culotte.

Le document US-A-3 461 872 propose de réaliser une culotte porte-couche, c'est-à-dire une culotte imperméable à l'eau destinée à recevoir une couche absorbante, à partir d'une feuille de mousse de matière plastique garnie sur tous les bords d'élastiques rapportés. Le but de l'utilisation de mousse de matière plastique pour cette culotte est d'empêcher le glissement, par rapport à la culotte, de la couche qui doit être placée de façon amovible dans la culotte, ainsi que de permettre le passage de l'air (vapeur) à travers la culotte. Ce document ne suggère pas d'assurer le serrage élastique à la taille et le serrage élastique aux passages des jambes sur une couche-culotte uniquement par l'utilisation de mousse élastique de matière plastique pour l'enveloppe extérieure et la feuille intérieure d'une couche-culotte.

Enfin, le document US-A-3 882 871 se rapporte à un article d'hygiène comprenant un porte-couche en textile, muni d'un élastique rapporté à la taille, une couche absorbante en matériau textile, et une toile textile hydrophobe destinée à venir se placer entre la couche et la peau de l'enfant, les trois éléments étant élastiques. Ce document ne suggère pas non plus les multiples fonctions assurées, sur la couche-culotte conforme à l'invention, par l'enveloppe extérieure et la feuille intérieure, grâce à leur constitution par de la mousse élastique de matière plastique.

La présente invention a pour objet une couche-culotte qui, tout en étant d'une fabrication plus simple que les couches-culottes à élastiques rapportés, assure à la fois une parfaite tenue et une bonne étanchéité sans provoquer un gonflement inesthétique et dont la pose correcte sur l'enfant n'exige pas la précision que nécessite les couches-culottes connues.

Telle que revendiquée, la couche-culotte conforme à l'invention, notamment pour enfants en bas âge, comprend une enveloppe extérieure pratiquement étanche à l'eau, une feuille intérieure perméable à l'eau, un coussin absorbant disposé entre ladite enveloppe extérieure et ladite feuille intérieure, et des moyens de fermeture fixés sur les deux bords latéraux opposés de la couche-culotte pour fermer cette dernière autour de l'enfant au niveau de la taille. Selon l'invention l'enveloppe extérieure et la feuille intérieure sont entièrement constituées par des mousses élastiques de matière plastique de manière à assurer un serrage élastique à la taille et aux passages pour les jambes, la mousse de l'enveloppe extérieure étant imperméable à l'eau, mais perméable à l'air et la mousse de la feuille intérieure étant perméable à l'eau et perméable à l'air.

Tout au moins la feuille intérieure est avantageusement constituée par une mousse de matière plastique au moins partiellement réticulée. En effet, la réticulation de la mousse provoque un éclatement des bulles de la mousse, ce qui donne à la mousse sa perméabilité.

De préférence, l'enveloppe extérieure est également constituée par une mousse au moins partiellement réticulée.

Par le degré de réticulation des mousses utilisées, il est donc possible de choisir la perméabilité de ces mousses aussi bien à l'eau qu'à l'air.

Une autre possibilité pour le choix de la perméabilité de l'enveloppe extérieure et de la feuille intérieure en mousse de matière plastique, de préférence au moins partiellement réticulée, consiste en l'épaisseur de cette enveloppe et de cette feuille. Il est ainsi en particulier possible de donner à la feuille extérieure une épaisseur telle qu'elle soit étanche à l'eau, mais perméable à l'air.

Bien entendu, il est également possible de faire intervenir à la fois le degré de réticulation de la mousse et l'épaisseur pour donner à l'enveloppe extérieure et à la feuille intérieure l'étanchéité à l'eau et la perméabilité à l'air recherchées.

En cas d'utilisation d'une même mousse de matière plastique pour l'enveloppe extérieure et la feuille intérieure, il est avantageux de donner à la feuille extérieure une épaisseur comprise entre 1 et 5 mm, de préférence entre 2 et 4 mm, et à la feuille intérieure une épaisseur comprise entre 0,5 et 1,5 mm, de préférence entre 0,8 et 1,2 mm.

La mousse de polyuréthane et la mousse de polyester ont, par exemple, donné d'excellents résultats.

De préférence, l'enveloppe extérieure et la feuille intérieure sont réunies par une ligne de soudure ou de collage périphérique.

Le coussin absorbant qui n'est généralement pas élastique est avantageusement fixé, au milieu seulement de la largeur de la couche-culotte, par une ou plusieurs lignes longitudinales de collage, à l'enveloppe extérieure ou à la feuille intérieure. L'enveloppe extérieure et la feuille intérieure conservent ainsi leur élasticité transversale, notamment au niveau de la ceinture.

En se référant au dessin annexé, on va décrire ci-après plus en détail un mode de réalisation illustratif et non limitatif d'une couche-culotte conforme à l'invention; sur le dessin:

la fig. 1 est une vue en plan d'une couche-culotte ouverte, à plat;

la fig. 2 est une coupe partielle suivant II–II de la fig. 1.

La couche-culotte illustrée par le dessin comprend, de façon connue, un coussin absorbant 1 disposé entre une feuille extérieure 2 et une feuille intérieure 3. Les deux feuilles 2 et 3 sont de forme générale sensiblement rectangulaire et présentent deux échancrures latérales opposées 4.

Le coussin absorbant 1 présente une forme correspondante en étant légèrement plus petit que les feuilles 2 et 3, de sorte que ses bords sont, sur toute la périphérie, légèrement en retrait par rapport au bord des feuilles 2 et 3.

La couche-culotte telle que décrite ci-dessus est de façon connue en soi utilisée de telle manière que la partie d'entrejambe 5 médiane, de largeur réduite, délimitée par les deux échancrures latérales opposées 4, soit placée entre les jambes de l'enfant, que l'une des parties d'extrémité plus larges ou partie avant 6 soit relevée sur le ventre de l'enfant et que l'autre partie d'extrémité plus large ou partie arrière 7 soit relevée sur la partie postérieure de l'enfant. Un dispositif de fermeture par adhésif 8 de type connu en soi, fixé sur les deux bords latéraux de la partie arrière 7, permet de fermer la couche-culotte autour de l'enfant au niveau de la ceinture, chacun des dispositifs de fermeture 8 étant collé sur la partie avant 6.

Selon la présente invention, les deux feuilles 2 et 3, au lieu d'être constituées, comme cela est usuel, la première par une feuille souple mais non élastique de matière plastique étanche à l'eau et la seconde par exemple par un voile de non-tissé souple, mais non élastique, perméable à l'eau, sont réalisées toutes les deux à partir de mousses de matière plastique élastiques en elles-mêmes. Les deux feuilles 2 et 3 peuvent être constituées par la même mousse de matière plastique, de préférence une mousse de matière plastique au moins partiellement réticulée, c'est-à-dire une mousse dont une partie au moins des bulles sont éclatées. Afin que, dans ce cas, la feuille intérieure 3 soit à la fois perméable à l'eau et à l'air et que la feuille extérieure 2 soit étanche à l'eau, mais de préférence perméable à l'air, la feuille intérieure 3 présente une épaisseur sensiblement plus faible que la feuille extérieure 2. A titre d'exemple, en cas d'utilisation de la même mousse de polyuréthane pour les deux feuilles 2 et 3, il est possible de donner à la feuille intérieure 3 une épaisseur comprise entre 0,5 et 1,5 mm et à la feuille extérieure 2 une épaisseur comprise entre 1 et 5 mm.

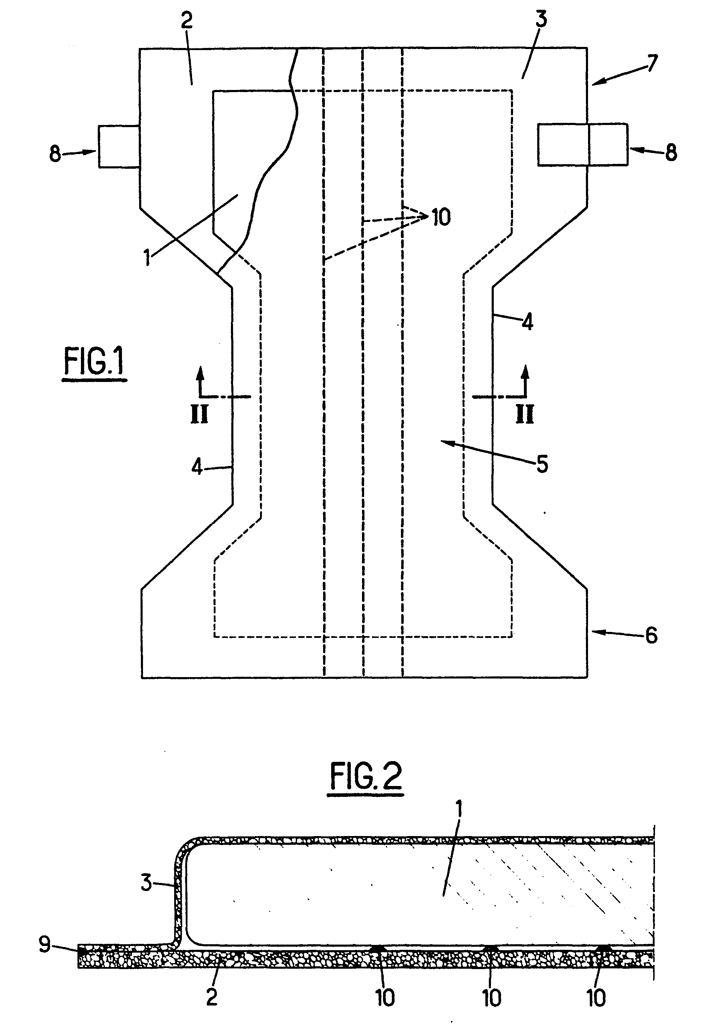

Les deux feuilles 2 et 3 sont reliées entre elles à leur bord, sur toute la périphérie, par une ligne de soudure 9 (fig. 2).

Le coussin absorbant 1 qui peut être de structure quelconque (cellulose ou tout autre produit absorbant) est fixé, lorsqu'il n'est pas élastique en lui-même, à la feuille extérieure 2 par trois lignes de colle 10 au milieu seulement de la largeur de la couche-culotte. Ces lignes de fixation 10 ont uniquement pour fonction de maintenir le coussin 1, sans réduire de façon notable l'élasticité transversale des feuilles 2 et 3 au niveau de la taille de la couche-culotte.

Il va de soi que l'invention qui réside dans l'utilisation de matières élastiques pour la feuille extérieure et la feuille intérieure d'une couche-culotte, feuilles entre lesquelles est disposé le coussin absorbant, est applicable à toutes les sortes de couches-culottes, c'est-à-dire aussi bien aux couches-culottes ouvertes telles que celle représentée qu'aux couches-culottes fermées en forme de slip.

Par ailleurs, au lieu d'utiliser la même mousse de matière plastique pour les deux feuilles et de donner à ces deux feuilles des épaisseurs différentes pour que l'une soit perméable à l'eau et l'autre étanche à l'eau, il serait possible également d'utiliser, pour les deux feuilles, des mousses différentes ou ayant des degrés de réticulation différents, voire de combiner les deux mesures (épaisseur et degré de réticulation) qui permettent d'agir sur la perméabilité.

La fabrication proprement dite de la couche-culotte peut s'effectuer suivant des procédés connus, de préférence des procédés faisant appel à deux bandes continues dans lesquelles les morceaux de feuille formant les différentes couches-culottes sont découpées en long ou en travers, après mise en place des coussins absorbants entre les bandes.

## Revendications

1. Couche-culotte, notamment pour enfants en bas âge, comprenant une enveloppe extérieure (2) pratiquement étanche à l'eau, une feuille intérieure (3) perméable à l'eau, un coussin absorbant (1) disposé entre ladite enveloppe extérieure et ladite feuille intérieure, et des moyens de fermeture (8) fixés sur les deux bords latéraux opposés de la couche-culotte pour fermer cette dernière autour de l'enfant au niveau de la taille, caractérisée par le fait que l'enveloppe extérieure (2) et la feuille intérieure (3) sont entièrement constituées par des mousses élastiques de matière plastique de manière à assurer un serrage élastique à la taille et aux passages pour les jambes, la mousse de l'enveloppe extérieure étant imperméable à l'eau, mais perméable à l'air et la mousse de la feuille intérieure étant perméable à l'eau et perméable à l'air.

2. Couche-culotte suivant la revendication 1, caractérisée par le fait que lesdites mousses de matière plastique sont au moins partiellement réticulées.

3. Couche-culotte suivant la revendication 1 ou 2, caractérisée par le fait que l'enveloppe extérieure est plus épaisse que la feuille intérieure.

4. Couche-culotte suivant la revendication 3, caractérisée par le fait que l'enveloppe extérieure présente une épaisseur comprise entre 1 et 5 mm, de préférence entre 2 et 4 mm, et que la feuille intérieure présente une épaisseur comprise entre 0,5 et 1,5 mm, de préférence entre 0,8 et 1,2 mm.

5. Couche-culotte suivant l'une quelconque des revendications 2 à 4, caractérisée par le fait que l'enveloppe extérieure est constituée par une mousse moins réticulée que la feuille intérieure.

6. Couche-culotte suivant l'une quelconque des revendications, caractérisée par le fait que l'enveloppe extérieure et la feuille intérieure sont fixées l'une à l'autre par une ligne de soudure ou de collage périphérique (9).

7. Couche-culotte suivant l'une quelconque des revendications précédentes, caractérisée par le fait que le coussin absorbant (1) est fixé, au milieu seulement de la largeur de la couche-culotte, par une ou plusieurs lignes longitudinales de collage (10) à l'enveloppe extérieure et à la feuille intérieure.

8. Couche-culotte suivant l'une quelconque des revendications précédentes, caractérisée par le fait que l'enveloppe extérieure et/ou la feuille intérieure sont constituée(s) par de la mousse de polyuréthane ou de polyester.

## Claims

1. Diaper, particularly for infants, comprising a practically watertight outer envelope (2), a water-permeable inner sheet (3), an absorbent pad (1) arranged between the said outer envelope and the said inner sheet, and means of closure (8) attached to the two opposing lateral edges of the diaper to close the latter around the child at the waist, characterised by the fact that outer envelope (2) and inner sheet (3) consist entirely of elastic foams of plastic material so as to ensure an elastic gripping at the waist and at the openings for the legs, the foam of the outer envelope being impermeable to water but permeable to air and the foam of the inner sheet being water-permeable and air-permeable.

2. Diaper according to claim 1, characterised by the fact that the said foams of plastic material are at least partially reticulated.

3. Diaper according to claim 1 or 2, characterised by the fact that the outer envelope is thicker than the inner sheet.

4. Diaper according to claim 3, characterised by the fact that the outer envelope has a thickness of between 1 and 5 mm, preferably between 2 and 4 mm, and that the inner sheet has a thickness of between 0.5 and 1.5 mm, preferably between 0.8 and 1.2 mm.

5. Diaper according to any of claims 2 to 4, characterised by the fact that the outer envelope consists of a foam less reticulated than the inner sheet.

6. Diaper according to any of the above claims, characterised by the fact that the outer envelope and the inner sheet are attached to one another by a peripheral welded or bonded line (9).

7. Diaper according to any of the above claims, characterised by the fact that absorbent pad (1) is attached, at the middle only of the width of the diaper, by one or several longitudinal bonding lines (10) to the outer envelope and to the inner sheet.

8. Diaper according to any of the above claims, characterised by the fact that the outer envelope and/or the inner sheet consist of polyurethane or polyester foam.

## Patentansprüche

1. Windelhöschen, insbesondere für Kleinkinder, das eine praktisch wasserdichte Aussenhülle (2), eine wasserdurchlässige Innenlage (3), ein saugfähiges Kissen (1), das zwischen der genannten Aussenhülle und der genannten Innenlage angeordnet ist und Verschlussmittel (8) aufweist, die an den beiden gegenüberliegenden Seitenrändern des Windelhöschens zum Verschliessen des letzteren um das Kind im Bereich der Taille befestigt sind, dadurch gekennzeichnet, dass die Aussenhülle (2) und die Innenlage (3) zur Gänze aus elastischen Schaumstoffen auf Kunststoffbasis bestehen der Art, dass ein elastischer Sitz in der Taille und bei den Beindurchtritten gewährleistet ist, wobei der Schaumstoff der Aussenhülle wasserundurchlässig, jedoch luftdurchlässig ist und wobei der Schaumstoff der Innenlage wasser- und luftdurchlässig ist.

2. Windelhöschen nach Anspruch 1, dadurch gekennzeichnet, dass die genannten Schaumstoffe auf Kunststoffbasis wenigstens teilweise vernetzt sind.

3. Windelhöschen nach Anspruch 1 oder 2, dadurch gekennzeichnet, dass die Aussenhülle dicker ist als die Innenlage.

4. Windelhöschen nach Anspruch 3, dadurch gekennzeichnet, dass die Aussenhülle eine Dicke zwischen 1 und 5 mm, vorzugsweise zwischen 2 und 4 mm und dass die Innenlage eine Dicke zwischen 0,5 und 1,5 mm, vorzugsweise zwischen 0,8 und 1,2 mm aufweist.

5. Windelhöschen nach einem der Ansprüche 2 bis 4, dadurch gekennzeichnet, dass die Aussenhülle aus einem Schaumstoff besteht, der weniger vernetzt ist als die Innenlage.

6. Windelhöschen nach einem der vorhergehenden Ansprüche, dadurch gekennzeichnet, dass die Aussenhülle und die Innenlage miteinander durch eine ringsherumlaufende Schweiss- oder Klebenaht (9) verbunden sind.

7. Windelhöschen nach einem der vorhergehenden Ansprüche, dadurch gekennzeichnet, dass das saugfähige Kissen (1) lediglich in der Mitte der Breite des Windelhöschens durch eine oder mehrere längslaufende Klebestreifen (10) an der Aussenhülle und der Innenlage befestigt ist.

8. Windelhöschen nach einem der vorhergehenden Ansprüche, dadurch gekennzeichnet, dass die Aussenhülle und/oder die Innenlage aus Polyurethan- oder Polyesterschaumstoff bestehen.

EP 0 098 983 B1

FIG.1

FIG.2

7